(19) **Europäisches Patentamt / European Patent Office / Office européen des brevets**

(11) **EP 4 692 298 A1**

(12) **EUROPEAN PATENT APPLICATION**
published in accordance with Art. 153(4) EPC

(43) Date of publication:
**11.02.2026 Bulletin 2026/07**

(21) Application number: **24778818.5**

(22) Date of filing: **16.02.2024**

(51) International Patent Classification (IPC):
***C12M 1/00*** (2006.01)  ***C12Q 1/02*** (2006.01)

(52) Cooperative Patent Classification (CPC):
**C12M 1/00; C12Q 1/02**

(86) International application number:
**PCT/JP2024/005555**

(87) International publication number:
**WO 2024/202673 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 JP 2023059403**

(71) Applicant: **Hitachi Plant Services Co., Ltd.**
**Tokyo 170-6034 (JP)**

(72) Inventors:
• **SHIBUYA, Keisuke**
**Tokyo 100-8280 (JP)**
• **OKA, Kenichiro**
**Tokyo 100-8280 (JP)**

(74) Representative: **MERH-IP Matias Erny Reichl Hoffmann**
**Patentanwälte PartG mbB**
**Paul-Heyse-Straße 29**
**80336 München (DE)**

(54) **CULTURE DATA COLLECTION DEVICE FOR CULTURE SCALE-UP AND CULTURE DATA COLLECTION METHOD FOR CULTURE SCALE-UP**

(57) The invention provides a culture scale-up culture data collection device and a culture scale-up culture data collection method that can prevent unexpected problems in productivity and quality after scale-up. The invention provides a culture scale-up culture data collection device (1) for searching for a scale-up condition of a cell culture device. The device has a first function related to a first explanatory variable associated with the cell culture device after scale-up, and a second function related to a second explanatory variable, which correlates with an objective variable but is not associated with the cell culture device after the scale-up. Both the first function and the second function are optimized for the objective variable.

[FIG. 4A]

EP 4 692 298 A1

## Description

Technical Field

The present invention relates to a culture scale-up culture data collection device and a culture scale-up culture data collection method. Specifically, the invention relates to a small culture device and a culture method for collecting data used for constructing a production process of culturing a large number of cells for producing a useful substance.

Background Art

Methods for producing useful substances by culturing cells of plants, microorganisms, animals, and the like are used in industries in various fields such as brewing, food, chemistry, and pharmaceutical products. For example, bio-pharmaceuticals such as antibody drugs contain a substance produced by animal cells as a main component. The substance is obtained by culturing animal cells and separating and purifying a target substance secreted in a culture solution.

In the construction of the production process for the useful substance, first, process conditions such as culture conditions such as temperature and pH are optimized on a small scale (several milliliters to several tens liters) in a laboratory scale culture experiment (laboratory experiment). Thereafter, in the construction of the production process for the useful substance, scale-up is performed so as to maintain quality of a product while maintaining production efficiency in a process optimized in the laboratory experiment in a desired production scale. As a scale-up method, scale-up using the law of similarity and scale-up using a design space have been known.

The scale-up based on the law of similarity is generally performed on the basis of a geometric similarity (all ratios between a structure and an arrangement of a stirring device are similar) and a constant power required for stirring per unit liquid volume. Specifically, a tank is designed such that the power required for the stirring, power consumption per unit liquid volume, a rotational speed of a stirring blade, a stirring blade diameter, a liquid discharge speed, a liquid circulation speed in a reactor, a stirring blade tip speed, and the Reynolds number are constant before and after the scale-up. However, it is difficult to perform the scale-up so as to satisfy all items at the same time, and thus, the scale-up is performed by paying attention to the fact that the most important items (items affecting productivity and quality) of stirring are satisfied.

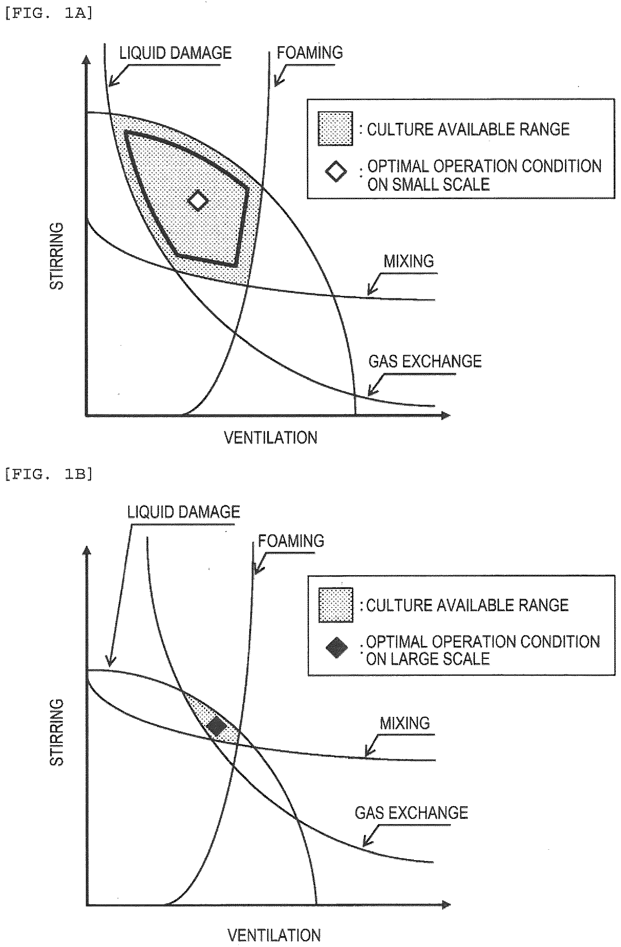

FIG. 1A is a conceptual diagram illustrating a concept of scale-up of a culture tank using a design space, and illustrates operation conditions on a small scale. FIG. 1B is a conceptual diagram illustrating a concept of scale-up of a culture tank using a design space, and illustrates operation conditions on a large scale (100 L or more).

As illustrated in FIG. 1A, in the scale-up using the design space, a condition range in which production can be performed is evaluated by a laboratory experiment centering on an optimum condition (optimum point) within a range in which culture can be performed and which is obtained by the laboratory experiment on a small scale. Thereafter, as illustrated in FIG. 1B, when a tank in a desired production scale is designed, a large-scale tank is designed by fluid analysis so that a region in which the production can be performed exists. As illustrated in FIGS. 1A and 1B, the range in which the culture can be performed is set to be wide on a small scale. However, the range in which the culture can be performed is often narrowed in the large scale due to the influence of factors that have not been evaluated in laboratory experiments.

Regarding the scale-up using the design space, for example, an invention described in PTL 1 has been proposed. The invention described in PTL 1 provides a computer implementation method for designing an experiment in a first scale for an organism so as to generate performance data of the first scale used when predicting performance of the organism in a larger second scale, and states that the method includes predetermined steps a to c. Among these steps, the step b is described as a step of determining a screening parameter of the first scale at least partially based on computer modeling of metabolism of the organism in the second scale.

Citation List

Patent Literature

PTL 1: JP2022-531464A

Summary of Invention

Technical Problem

The tank design for which the scale-up method is used as described above has the following problems.

Although a large number of culture experiments are performed in a laboratory scale to optimize the process, culture experiment data necessary for scale-up is collected again in the scale-up, and thus, a period required for the construction of a production process from the laboratory scale tends to be long.

In addition to the above, when culture is performed in a culture process (production process) after scale-up of a culture process optimized in a laboratory scale is performed, events with different productivity and quality may occur.

In the step b of the invention described in PTL 1, what is considered when the screening parameter of the first scale is determined is a simulation result after the scale-up, and is different from a variable that cannot be operated (changed) after the scale-up. Therefore, in the invention described in PTL 1, it is a problem to prevent an unexpected problem in productivity and quality after the scale-up.

The invention has been made in view of the circumstances in the related art. An object of the invention is to provide a culture scale-up culture data collection device and a culture scale-up culture data collection method that can prevent unexpected problems in productivity and quality after scale-up.

Solution to Problem

The invention provides a culture scale-up culture data collection device (1) for searching for a scale-up condition of a cell culture device. The culture scale-up culture data collection device has a first function related to a first explanatory variable associated with the cell culture device after scale-up, and a second function related to a second explanatory variable, which correlates with an objective variable but is not associated with the cell culture device after the scale-up. Both the first function and the second function are optimized for the objective variable.

Advantageous Effects of Invention

The invention can provide a culture scale-up culture data collection device and a culture scale-up culture data collection method that can prevent unexpected problems in productivity and quality after scale-up.

Brief Description of Drawings

[FIG. 1A] FIG. 1A is a conceptual diagram illustrating a concept of scale-up of a culture tank using a design space, and illustrates operation conditions on a small scale.

[FIG. 1B] FIG. 1B is a conceptual diagram illustrating a concept of scale-up of a culture tank using a design space, and illustrates operation conditions on a large scale.

[FIG. 2] FIG. 2 is a diagram illustrating a correlation mechanism between setting and design parameters and quality and productivity parameters.

[FIG. 3] FIG. 3 is a conceptual diagram illustrating a concept of a function of a small-scale culture tank.

[FIG. 4A] FIG. 4A is a diagram illustrating a configuration of a small-scale culture tank according to the related art for evaluating parameters after scale-up.

[FIG. 4B] FIG. 4B is a diagram illustrating a configuration of a culture scale-up culture data collection device according to the present embodiment for evaluating the parameters after the scale-up.

[FIG. 5] FIG. 5 is a flowchart illustrating contents of a culture scale-up culture data collection method according to the present embodiment.

[FIG. 6] FIG. 6 is a diagram illustrating a device configuration of a bubble influence evaluation experiment using a culture tank of 1 L.

[FIG. 7] FIG. 7 is a graph illustrating a growth curve in each in-liquid ventilation. In the figure, a horizontal axis represents the number of culture days (day), and a vertical axis represents a viable cell number density ($\times 10^6$ cells/mL).

[FIG. 8] FIG. 8 is a graph illustrating a relationship between a total ventilation volume and a cell loss number density. In the figure, a horizontal axis represents a total ventilation volume (mL), and a vertical axis represents a cell loss number density ($\times 10^6$ cells/mL).

[FIG. 9] FIG. 9 is a graph illustrating a relationship between the number of culture days and the viable cell number density, and illustrates cell growth curves before the scale-up (3 L) and after the scale-up (100 L). In the figure, a horizontal axis represents the number of culture days (day), and a vertical axis represents a viable cell number density ($\times 10^6$ cells/mL).

[FIG. 10] FIG. 10 is a graph illustrating a relationship between measured values of a lactic acid (Lac) production rate and a glutamine (Gln) consumption rate and estimated values (models) obtained by calculation based on shear force distribution when 100 L culture is performed. In the figure, a horizontal axis represents estimated values, and a vertical axis represents measured values.

Description of Embodiments

[Culture Scale-up Culture Data Collection Device]

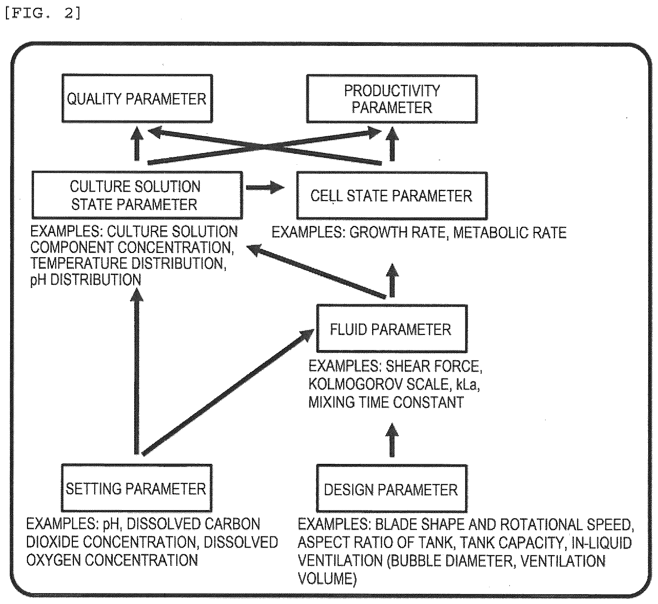

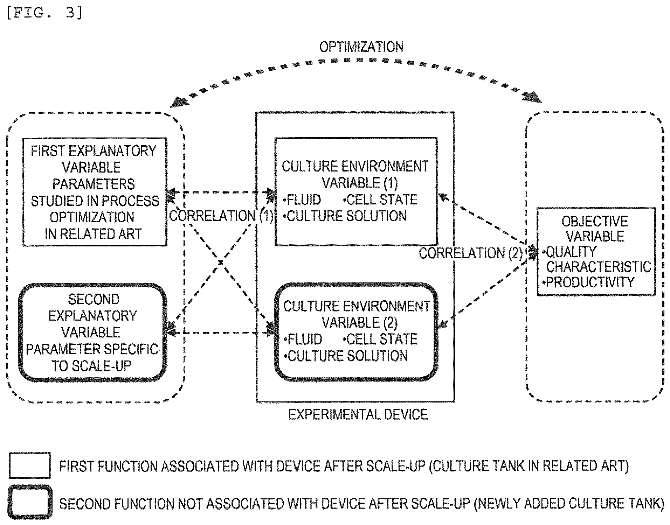

Hereinafter, an embodiment of the invention will be described in detail with reference to the drawings as appropriate. In the drawings to be referred to, FIG. 2 is a diagram illustrating a correlation mechanism between setting and design parameters and quality and productivity parameters. FIG. 3 is a conceptual diagram illustrating a concept of a function of a small-scale culture tank.

FIGS. 2 and 3 illustrate a correlation between the culture conditions and the productivity and quality of a product. As illustrated in FIGS. 2 and 3, parameters studied in the optimization of the culture process in the related art are mainly pH, dissolved carbon dioxide ($DCO_2$) concentration, dissolved oxygen (DO) concentration, temperature, osmotic pressure, and the like related to a first explanatory variable. That is, the first explanatory variable is a setting parameter for setting and controlling culture conditions in a cell culture device after scale-up.

In the scale-up according to the related art, an appropriate culture condition and an allowable range thereof in a laboratory experiment are determined using the setting parameters. In the desired large scale-up, whether an average value of the setting parameters in the tank, or a maximum value, or a minimum value of distribution of the setting parameters falls within the allowable range determined on a small scale is evaluated. However, among parameters that affect the productivity and quality of products, parameters specific to scale-up, which are not taken into consideration in the related art, are also present. Examples of the parameters specific to scale-up include, but are not limited to, shear force distribution, nutrient component concentration distribution, metabolite concentration distribution, waste product concentration distribution, Kolmogorov scale, kLa, and a mixing time constant. These parameters specific to scale-up are mainly fluid parameters, and are affected by design parameters such as a blade shape and a rotational speed, an aspect ratio of a tank, a tank capacity, and an in-liquid ventilation (a bubble diameter and a ventilation volume). The fluid parameters and the design parameters cannot be set in the cell culture device after the scale-up. In the present embodiment, among the parameters that cannot be set in the cell culture device after the scale-up, the fluid parameters are treated as a second explanatory variable. As illustrated in FIGS. 2 and 3, the setting parameter (first explanatory variable) and the fluid parameter (second explanatory variable) are correlated with each other (correlation (1) in FIG. 3), and affect a culture solution state parameter (a culture environment variable (1)) and a cell state parameter (a culture environment variable (2)). Both the culture environment variable (1) and the culture environment variable (2) relate to a fluid, a cell state, and a culture solution. Examples of the culture solution state parameter include a culture solution component concentration, temperature distribution, and pH distribution. Examples of the cell state parameter include a growth rate and a metabolic rate. As illustrated in FIGS. 2 and 3, the culture solution state parameter and the cell state parameter are correlated with each other (correlation (2) in FIG. 3), and affect an objective variable such as quality characteristics (quality parameters) and productivity (productivity parameters).

Therefore, it is necessary to evaluate not only the first explanatory variable but also the parameter specific to scale-up (second explanatory variable) in the small-scale culture experiment (laboratory experiment), but setting and control items of the culture device in the small-scale culture experiment according to the related art are the same as setting and control items of the culture tank after the scale-up. Therefore, for items that cannot be set and controlled after the scale-up, the influence on the productivity and quality of the products cannot be evaluated in the small-scale culture device according to the related art.

In contrast, in the present embodiment, in the small-scale culture experiment, in addition to the parameter (first explanatory variable) evaluated in the related art, the parameter (second explanatory variable) specific to the scale-up is evaluated, and the scale-up is performed using these pieces of evaluation data. Therefore, the inconsistency in productivity and quality occurring after the scale-up can be prevented.

In the present embodiment, a configuration example of a small-scale culture tank (culture scale-up culture data collection device) for searching for a scale-up condition of a cell culture device is presented.

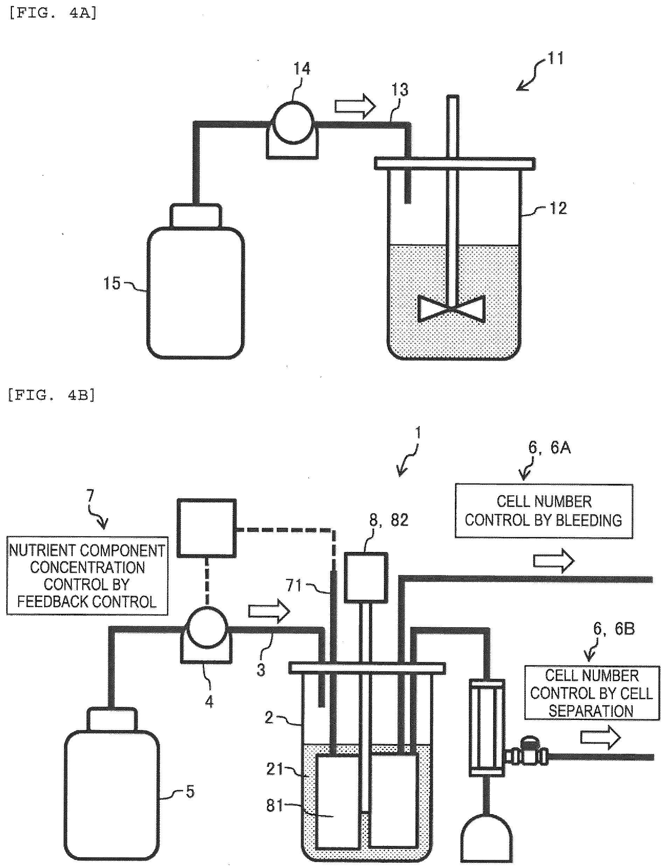

FIG. 4A is a diagram illustrating a configuration of the small-scale culture tank according to the related art for evaluating parameters after scale-up. FIG. 4B is a diagram illustrating a configuration of a culture scale-up culture data collection device 1 according to the present embodiment (hereinafter, may be abbreviated as "the present device 1") for evaluating parameters after the scale-up.

As illustrated in FIG. 4A, a small-scale (data collection) culture tank 11 according to the related art has the same structure as a large-scale (production) culture tank. The small-scale culture tank 11 according to the related art includes, for example, a culture tank 12 and a medium tank 15 connected with each other via an addition tube 13 and an addition pump 14.

In contrast, as illustrated in FIG. 4B, the present device 1 has a first function related to the first explanatory variable associated with the cell culture device after the scale-up, and a second function related to the second explanatory variable that correlates with the objective variable but is not associated with the cell culture device after the scale-up. Then, the present device 1 optimizes both the first function and the second function for the objective variable (searches for the most preferable scale-up condition).

As illustrated in FIG. 4B, specific configuration examples of the present device 1 include, in addition to a culture tank 2 and a medium tank 5 connected thereto via an addition tube 3 and an addition pump 4, a cell number control unit 6 that controls the cell number, a nutrient component concentration control unit 7 that controls a nutrient component concentration, and a shear force control unit 8 that controls a shear force. Further, a specific configuration example of the present device 1 includes a waste product concentration control unit (not illustrated).

Examples of the cell number control unit 6 include cell number control by a cell bleeding (cell extraction) device 6A (cell removal). That is, the cell bleeding device 6A reduces the number of cells in the culture tank 2 by extracting cells together with a culture solution 21.

Examples of the cell number control unit 6 include cell number control by a cell separation device 6B (an improvement in cell number concentration). That is, the cell number per unit volume of the culture solution 21 can be increased by removing only the culture solution 21 from the culture tank 2 while leaving the cells by the cell separation device 6B and returning the remaining cells to the culture tank 2. The cell separation device 6B is preferably of a gravitational settling type or a hollow fiber membrane separation type. These types can be used to preferably separate cells.

As the nutrient component concentration control unit 7, for example, a concentration or the like of nutrient components in the culture solution 21 is measured using a medium component analysis device (not illustrated) to be described below, which is connected to a sampling tube 71 provided in the culture solution 21 in the culture tank 2, feedback control is performed by an operation device (not illustrated) based on a measurement result, and the addition pump 4 and a culture solution extraction pump (not illustrated) are controlled to add a liquid medium from the medium tank 5 to the culture tank 2, thereby controlling a nutrient component concentration of the culture solution 21.

As the waste product concentration control unit (not illustrated), for example, a concentration or the like of the waste product in the culture solution 21 is measured using a medium component analysis device (not illustrated) to be described below, which is connected to the sampling tube 71 provided in the culture solution 21 in the culture tank 2, feedback control is performed by an operation device (not illustrated) based on the, measurement result, and the addition pump 4 and the culture solution extraction pump (not illustrated) are controlled to add a liquid medium from the medium tank 5 to the culture tank 2, thereby controlling a waste product concentration of the culture solution 21. The cell bleeding device 6A or the cell separation device 6B described above can be used as one waste product concentration control unit.

The present device 1 includes the cell number control unit 6, so that the cell number is kept constant. In addition, the present device 1 includes the nutrient component concentration control unit 7 and the waste product concentration control unit, so that the nutrient component concentration and the waste product concentration can be kept constant. Therefore, according to the present embodiment, the cell state, the nutritional state, and the like are constant, and therefore, the obtained data is more reliable.

Examples of the shear force control unit 8 include a motor 82 that rotates a stirring blade 81 and a control device (not illustrated) that controls a rotational speed of the motor 82. Examples of the shear force control unit 8 include a high shear force stirring blade to be described below.

That is, in order to control the second function, the present device 1 preferably includes at least one of the cell separation device 6B, the cell bleeding device 6A, the medium component analysis device (not illustrated), a medium addition device (such as the addition pump 4 and the medium tank 5) that performs feedback control based on values of the medium component, and the shear force control unit 8 such as a high shear force stirring blade.

The cell number control unit 6, the nutrient component concentration control unit 7, the waste product concentration control unit, and the shear force control unit 8 correspond to the second function related to the second explanatory variable, and optimize both the first function related to the first explanatory variable such as temperature or pH and the second function for the objective variable. Accordingly, the present device 1 can prevent unexpected problems in productivity and quality after scale-up.

[Culture Scale-up Culture Data Collection Method]

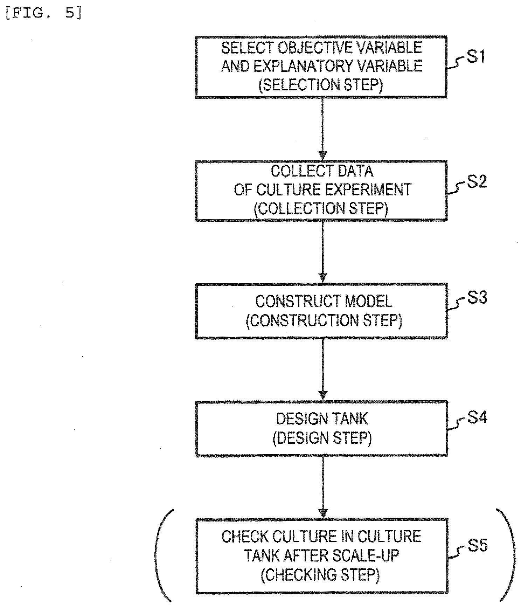

Next, a procedure of scale-up in the present embodiment is illustrated in FIG. 5. FIG. 5 is a flowchart illustrating contents of a culture scale-up culture data collection method according to the present embodiment (hereinafter may be abbreviated as "the present method").

The present method is a culture scale-up culture data collection method for searching for scale-up conditions of a cell culture device. As illustrated in FIG. 5, the present method includes a selection step S1, a collection step S2, a construction step S3, and a design step S4. In addition, the present method preferably includes a checking step S5 after the design step S4.

Hereinafter, the items of the present method will be described in detail.

1. Selection of Objective Variable and Explanatory Variable

(Selection Step S1)

In the selection step S1, the objective variable, the first explanatory variable associated with the cell culture device after the scale-up, and the second explanatory variable correlated with the objective variable but not associated with the cell culture device after the scale-up are selected.

The objective variable is productivity, quality, and the like of the substance to be produced, and examples thereof include, but are not limited to, a concentration of the product, a total yield of the product, and a purity of the product. The objective variable does not change before and after the scale-up.

The explanatory variable is divided into a variable (first explanatory variable) that does not change before and after the scale-up and a variable (second explanatory variable) that can be set and controlled before the scale-up and is not set and controlled after the scale-up.

The first explanatory variable is a parameter set and controlled after the scale-up, and is, for example, temperature, pH, DO, $DCO_2$, or osmotic pressure as described above. Therefore, the first function related to the first explanatory variable is preferably a control device that controls at least one of the first explanatory variables.

As described above, the second explanatory variable is, for example, the shear force, the Kolmogorov scale, the nutrient component concentration distribution, or intra-layer distribution in the above items including the first explanatory variable. Therefore, the second function related to the second explanatory variable is preferably a control device that controls at least one of the second explanatory variables.

2. Data Collection in Culture Experiment (Collection Step S2)

In the collection step S2, a cell culture experiment is performed, based on the first explanatory variable and the second explanatory variable selected in the selection step S1, using cells to be used, and data of at least one of a shear force, a nutrient component concentration, a waste product concentration, and a viable cell number concentration is collected. The present device 1 includes a data collection device (not illustrated) such as a hard disk drive (HDD) or a solid state drive (SSD), and the collected data is collected (stored) in the data collection device. In this cell culture experiment, each parameter of the first explanatory variable is changed as in the related art, and the objective variable under that condition is measured. On the other hand, the second explanatory variable is a parameter that is not measured in the small-scale culture experiment according to the related art in the culture process optimization. An example of a measurement method of the second explanatory variable and a device function for the measurement will be described below with reference to FIG. 4B.

(Shear Force)

The shear force is distributed in the culture tank 2 and is difficult to directly measure. Therefore, for the shear force, the shear force distribution when the stirring rotational speed is changed is quantified by fluid analysis (simulation) based on a structure of the culture tank 2 used in the culture experiment. An inclined paddle type stirring blade is often used in the related art, but in this case, the magnitude of the shear force after scale-up is not reached even if the rotational speed is maximized, and therefore, it is desirable to increase a diameter of the stirring blade and use a vertically long flat paddle. The vertically long flat paddle having a large stirring blade diameter can be exemplified as one of the high shear force stirring blades.

(Nutrient Component Concentration)

The nutrient component concentration is controlled by measuring a nutrient component concentration of the culture solution 21 and adding, based on the measured value, a medium containing nutrients to reach a set value. Examples of nutrient components include glucose, glutamine, and glutamic acid. It is preferable to measure and control the nutrient component concentration of at least one of these nutrient components. A method for measuring the nutrient component concentration can be performed by sampling the culture solution 21 and using a medium component analysis device such as HPLC or LC-MS, or by in-line sensing using Raman spectroscopy. Then, the measured value is input to an operation device (not illustrated), and the addition pump 4 and the culture solution extraction pump (not illustrated) are operated to control the nutrient component concentration.

(Waste Product Concentration)

The waste product concentration is controlled by measuring a waste product concentration of the culture solution 21 and removing, based on the measured value, a waste product and/or a culture medium containing the waste product to reach a set value. Examples of the waste product include lactic acid and ammonia. It is preferable to measure and control the waste product concentration of at least one of these waste products. A method for measuring the waste product concentration can be performed by sampling the culture solution 21 and using a medium component analysis device such as HPLC or LC-MS, or by in-line sensing using Raman spectroscopy. Then, the measured value is input to an operation device (not illustrated), and the addition pump 4 and the culture solution extraction pump (not illustrated) are operated to control the waste product concentration.

(Viable Cell Number Concentration)

The viable cell number concentration is controlled by measuring a viable cell number concentration of the culture solution 21 and controlling, based on the measured value, the viable cell number concentration to reach a set value. When the viable cell concentration is higher than the set value, the culture solution 21 containing the viable cells is extracted by the cell bleeding device 6A, and the concentration is lowered by adding an equal amount of medium. When the viable cell number concentration is lower than the set value, the culture is continued until the number of viable cells reaches the set viable cell number. Further, the number of cells per unit volume of the culture solution 21 can be increased by removing only the medium while leaving the cells by the cell separation device 6B and returning the remaining cells to the culture solution 21. The viable cell number can be measured by sampling the culture solution 21 and counting the cell number using a microscope or an image, or may be counted in-line using capacitance or turbidity.

In order to collect data and optimize a process in a small-scale culture experiment, it is necessary to evaluate a condition in which each first explanatory variable and each second explanatory variable are combined. The number of experiments for evaluation depends on the number of items of each explanatory variable, and increases exponentially with respect to the number of items. When there are a large number of selected explanatory variables, a very large number of cell culture experiments have to be performed, and the time required for evaluation becomes very long. In order to solve this problem, it is desirable to arrange small-scale culture tanks in parallel and simultaneously perform evaluation under a plurality of culture conditions. In order to reduce the cost of consumables and the like required for the experiment, the number of experiments can be reduced by an experimental design method or Bayesian optimization.

(Bayesian Optimization)

In Bayesian optimization, a cell culture experiment is performed for a combination of the first explanatory variable and the second explanatory variable, an objective variable such as productivity is actually measured, and the following condition is presented so as to maximize (or minimize) the objective variable according to a Bayesian optimization method based on the information. In Bayesian optimization, a process of performing culture under the conditions, measuring the objective variable, and presenting the following conditions is repeated. In this cycle, a time point when there is no variation in the objective variable and the explanatory variable is the optimum point. When there are a large number of explanatory variables, the number of cell culture experiments can be significantly reduced by using Bayesian optimization.

3. Model Construction (Construction Step S3)

A model for the scale-up is constructed based on the data (culture data) collected in 2. in the construction step S3.

The data on the nutrient component concentration and the waste product concentration can be modeled by fitting to a Monod equation. In addition, the viable cell number concentration, the shear force, and the like can be formulated based on empirical rules.

In addition to the above method, when the process is optimized by Bayesian optimization, the periphery of the optimal point can be modeled with high accuracy, and thus this may be used as a model.

4. Tank Design (Design Step S4)

In the design step S4, at least one of productivity and quality after the scale-up is evaluated using the model constructed in 3. and fluid analysis, and design of the culture tank 2 is performed.

In the culture tank 2, each explanatory variable has distribution. The productivity and quality at each local point can be evaluated based on the model constructed in 3. and the distribution of various variables analyzed by fluid analysis, and then integrated over the entire cell culture tank, so that the cell culture tank after the scale-up is evaluated quantitatively. By utilizing the simulation, an appropriate structure of the cell culture tank after the scale-up can be determined.

In the small-scale culture tank, the distribution of the concentration or the like in the tank is small, and therefore, a value measured at one point in the tank is regarded as an average value. On the other hand, in a large-scale culture tank, concentration distribution in the tank occurs, and the influence of this distribution may not be negligible. In addition, the concentration distribution in the tank refers to a ratio of (a width of) each average value in the tank in average values of explanatory variables in divided spaces obtained by dividing the space in the tank.

5. Evaluation and Checking in Culture Tank after Scale-up

(Checking Step S5)

In the checking step S5, the cell culture tank (large-scale culture tank) designed in 4. is constructed, and whether a desired performance is satisfied is checked according to a cell culture experiment using the cell culture tank.

In the present embodiment, a culture device capable of evaluating parameters that have not been evaluated in a small-scale culture experiment in the related art is provided and data is collected, so that scale-up can be performed in consideration of characteristic parameters for the scale-up. In a data collection device according to the related art, the device configuration (functions such as temperature control and pH control) does not change before and after the scale-up. However, in the present embodiment, a device (second function) not associated with the culture device after the scale-up is associated with the small-scale culture device.

According to the culture scale-up culture data collection device and the culture scale-up culture data collection method according to the present embodiment, in addition to the culture conditions in the related art, it is possible to prevent (reduce) unexpected problems in productivity and quality before and after the scale-up by evaluating, by experiments, parameters that affect an objective variable that becomes apparent for the first time due to the scale-up. In the present embodiment, the number of explanatory variables increases in the experimental data collection, and therefore, the number of experiments required for optimization can be reduced, and the time and cost required for data collection can be reduced by using a method such as Bayesian optimization.

Examples

The culture scale-up culture data collection device and the culture scale-up culture data collection method according to the present embodiment will be specifically described with reference to Examples.

<<Scale-up from 3 L to 100 L using CHO Cells>>

Culture scale-up was performed on the Chinese hamster ovary cell (CHO cell) line using the present method described above. Details will be described below.

[1] Selection of Objective Variable and Explanatory Variable

Temperature, pH, and dissolved oxygen (DO) were selected as the first explanatory variables, and a shear force was selected as the second explanatory variable.

[2] Data Collection in Culture Experiment

The following culture experiment was performed in a 1 L or 3 L culture tank for the explanatory variables selected in [1] .

<Cells and Medium>

In the culture experiment, CHO cells (CRL-9606 cells) (both adhesion culture/floating culture) were purchased from American Type Culture Collection (ATCC) and used. The medium used was a medium obtained by supplementing a Ham's F12 basal medium with fetal bovine serum (FBS) (final concentration: 10%) and penicillin and streptomycin which are antibiotics.

<Preparation of Floating Cells (Adaptation from Adhering Cells to Floating Cells)>

CHO cells cultured in an adhering state using a culture flask were detached with trypsin. Next, the trypsin solution was removed by centrifugation (room temperature, $500 \times g$, 5 minutes), and then dilution was performed with a Ham's F12 medium. Next, using a spinner flask, the cells were cultured with stirring at a cell concentration of $1 \times 10^5$ cells/ml and a volume of 100 mL in an incubator (37°C, 5% $CO_2$, humidity of 90% or more). In this study, cells in which adherent cells were adapted to floating cells by the above procedure were used.

<Culture Operation>

Culture was performed using a 1 L or 3 L culture tank under the culture conditions described in the previous section. A seeding density was $1 \times 10^5$ cells/mL, and during the culture, the dissolved oxygen, the pH, and the temperature were controlled under various conditions. Sterile sampling was performed one to two times per day, and culture solution component analysis was performed.

<Analysis of Culture Solution Components>

Based on the sampled culture solution, (1) the viable cell number and (2) medium components (glucose, glutamine, lactic acid, and ammonia) were quantified. An analysis method is described below.

(1) Counting of Viable Cells

The viable cell number was measured using Vi-CELL (Beckman Coulter, Inc.), which is a device for determining whether a cell is viable or dead. The sampled culture solution was set in Vi-CELL, viable cells and dead cells were distinguished by a trypan blue staining method, image data of the cells was acquired, and a value of the viable cell number was obtained by automatic counting.

(2) Medium Component Analysis (Glucose, Glutamine, Lactic Acid, and Ammonia)

Glucose, glutamine, lactic acid, and ammonia in the culture solution were measured using Bioplofile 100 plus (Nova) .

<Experiment of Evaluating Influence of Bubbles on Cell Metabolism>

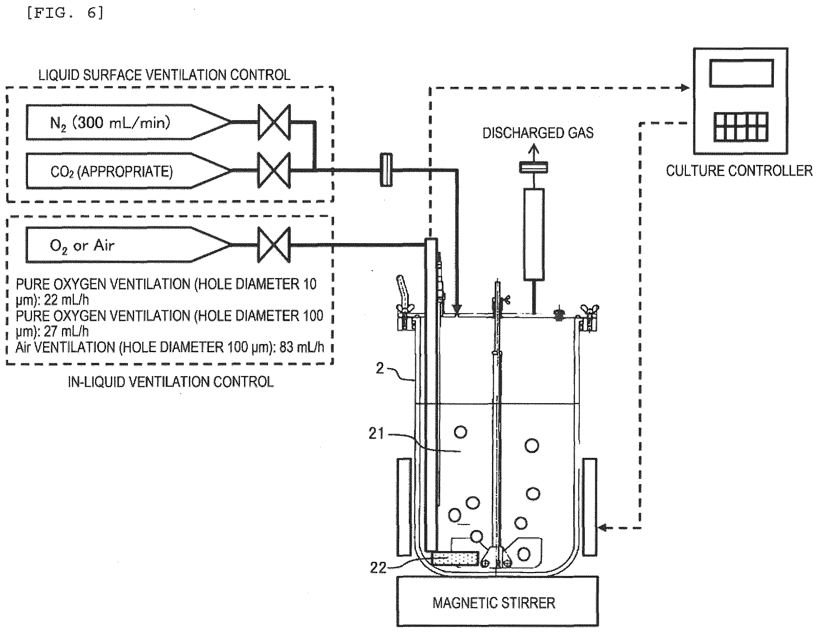

In an experiment of evaluating an influence of bubbles on cell metabolism, the culture tank 2 of 1 L was used. FIG. 6 is a diagram illustrating a device configuration of the bubble influence evaluation experiment using the culture tank 2 of 1 L. In order to examine the influence of bubbles, it is necessary to supply bubbles into the culture solution 21 while maintaining dissolved oxygen constant. Therefore, in the present culture experiment, the oxygen in the culture solution 21 was forcibly removed from a liquid surface by continuously blowing nitrogen (300 mL/min) onto the liquid surface, and bubbles were supplied in such a manner as to compensate for the degassing by the in-liquid ventilation. In the water test, it was found that there was no difference in the dissolved oxygen concentration between a vicinity of the liquid surface (1 cm below the liquid surface) and a bottom portion of the culture tank 2. In the liquid surface ventilation, in addition to nitrogen, carbon dioxide was appropriately mixed to maintain pH. In the in-liquid ventilation, Air ventilation or pure oxygen ventilation was performed to investigate the influence of the type of gas. The ventilation was performed at 22 mL/h in the case of the pure oxygen ventilation with a sparger 22 having a hole diameter of 10 $\mu$m, the ventilation was performed at 27 mL/h in the case of the pure oxygen ventilation with a sparger 22 having a hole diameter of 100 $\mu$m, and the ventilation was performed at 83 mL/h in the case of the Air ventilation with the sparger 22 having a hole diameter of 100 $\mu$m. In the present culture experiment, the culture experiment was performed under the condition that the shear force distribution was 0.5 Pa or less so as not to be influenced by the shear force distribution due to stirring.

(Results of Bubble Diameter)

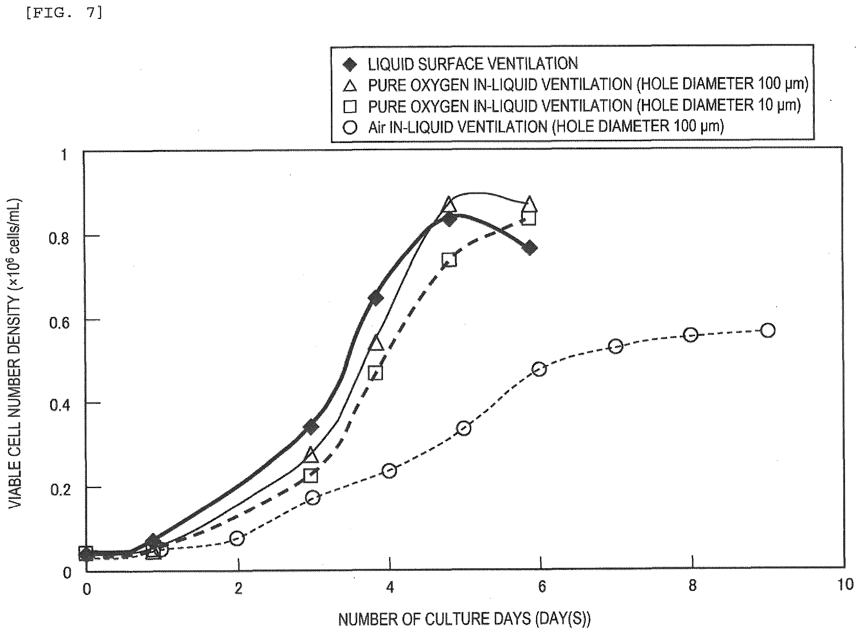

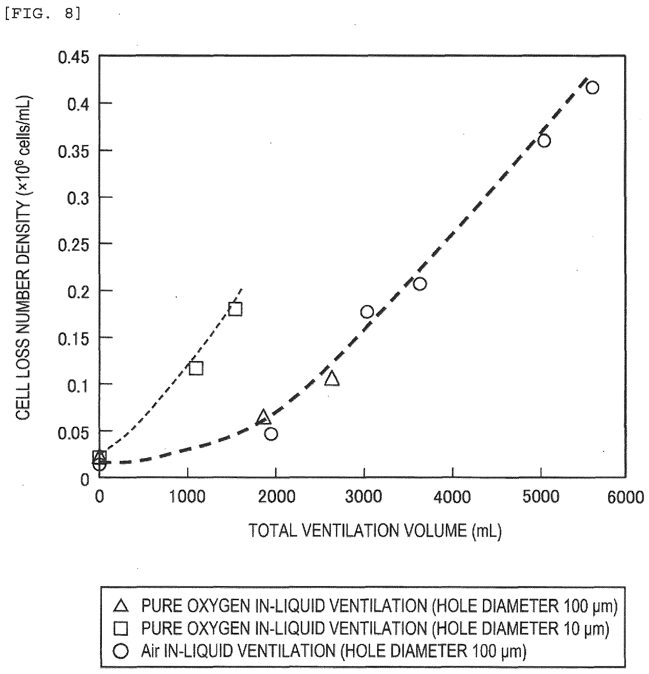

In the culture tank 2 of 1 L, the in-liquid ventilation was performed using the spargers 22 having different hole diameters, and the influence of bubbles on cells was evaluated. As described above, two types of in-liquid ventilation conditions, the pure oxygen ventilation and the Air ventilation, were performed. FIG. 7 is a graph illustrating a growth curve in each in-liquid ventilation. As illustrated in FIG. 7, in the in-liquid ventilation, the growth of cells is slow compared with only the liquid surface ventilation, and the reached cell number density tends to be low. In particular, in the Air in-liquid ventilation, the growth rate was 0.031 h$^{-1}$ (only in the liquid surface ventilation; 0.036 h$^{-1}$) , and the reached cell number was $0.55 \times 10^6$ cells/mL (only in the liquid surface ventilation; $0.83 \times 10^6$ cells/mL), and significant growth inhibition occurred.
In order to grasp a relationship between the reduction in the cell number due to the in-liquid ventilation and a ventilation volume, the cell number obtained by subtracting the cell number in the in-liquid ventilation from the cell number in the liquid surface ventilation at each time point was defined as the cell loss number, and a relationship between the cell loss number and a total ventilation volume at which the ventilation is performed from the start of culture to each time point was examined. FIG. 8 is a graph illustrating the relationship between the total ventilation volume and the cell loss number. However, the graph illustrated in FIG. 8 was calculated according to the cell number in a logarithmic growth phase (from day 1 to day 4 of the culture) in order to avoid the influence of nutrient depletion in the late stage of the culture. As illustrated in FIG. 8, there is a strong correlation between the total ventilation volume and the cell loss number, and the spargers 22 having the same hole diameter are on the same curve regardless of the type of gas (pure oxygen and Air). On the other hand, the correlation between the total ventilation volume and the cell loss number changes depending on the hole diameter of the sparger 22, and it was found from the results of the present culture experiment that the cell loss number for the same ventilation volume increases as the hole diameter decreases (10 $\mu$m). This phenomenon is presumed to increase the cell loss number because the cells are captured by the bubbles in the process of the bubbles rising from the sparger 22 present at the bottom portion of the culture tank 2, float up to the culture liquid surface, and adhere to a wall around the culture liquid surface, or the cells are also disrupted when the bubbles disappear. In addition, it is considered that the sparger 22 having a small hole diameter (hole diameter 10 $\mu$m) had a larger number of bubbles as the bubble diameter decreased, and thus the cell loss number was larger.

[3] Model Construction

The culture conditions optimized based on the data collected in [2] were temperature: 37°C, pH: 7.2, and DO: 2.7 mg/L. At this time, it was found that there is a correlation between the shear force and the metabolic rate. A model for estimating the metabolic rate based on the shear force for scale-up design was constructed as follows.

(Estimation of Operation Condition and Characteristic Quantity of 100 L Pilot Culture Tank)

In order to determine an operation condition in 100 L culture, a culture performance index (lactic acid production rate, specific growth rate, and the like) was formulated as a function of a shear force distribution. The culture performance index can be expressed by the formula (1) in which J represents the culture performance index, $F_i$ represents a volume fraction for each shear force range, and $c_i$ represents a weighting factor for each shear force range.
[Math. 1]

$$J \cong \sum_{i=1}^{N+1} c_i \cdot F_i \qquad \text{FORMULA (1)}$$

Then, when the weighting factor ci was determined so as to minimize the formula (2) based on shear force distribution in the fed-batch culture of 1 L and 3 L and data of a lactic acid production rate and a glutamine consumption rate at that time, the formulas (3) and (4) were obtained. Note that $\varphi$ in the formula (2) is an objective function for fitting to an experiment.
[Math. 2]

$$\phi = \sum_k \left| J^{(k)} - \sum_{i=1}^{N+1} c_i \cdot F_i^{(k)} \right| \qquad \text{FORMULA (2)}$$

[Math. 3]
(LACTIC ACID PRODUCTION RATE)

$$J_a = -0.0126 \cdot \langle 0,1.7 \rangle + 0.0027 \cdot \langle 1.7,8.0 \rangle - 0.0387 \cdot \langle 8.0,12.8 \rangle - 0.0065 \cdot \langle 12.8,+\infty \rangle \quad \text{FORMULA (3)}$$

[Math. 4]
(GLUTAMINE CONSUMPTION RATE)

$$J_h = -0.012 \cdot \langle 0,3.2 \rangle - 0.041 \cdot \langle 3.2,15.2 \rangle - 0.022 \cdot \langle 15.2,+\infty \rangle \qquad \text{FORMULA (4)}$$

[4] Tank Design

The stirring rotational speed was changed in a 100 L culture tank, and a point at which metabolism occurred under optimum conditions in 1 L and 3 L culture was searched based on the model of [3]. As a result, the stirring rotational speed in the 100 L culture tank was determined to be 75 rpm.

[5] Checking of Culture in Culture Tank after Scale-up

In the culture process optimization using the small-scale culture tank, when the shear force was included in the parameters, the optimum shear force was a shear force of 1 Pa or more, which was 90% or more of the tank in volume ratio.

This is called a high shear force. On the other hand, as in the related art, in the optimization of the culture process in which the shear force was not included as a parameter, the stirring conditions are stirring conditions (blade shape pitched paddle, 100 rpm) according to the related art, and the average shear force in the tank in that case was less than 1 Pa. This is called a low shear force.

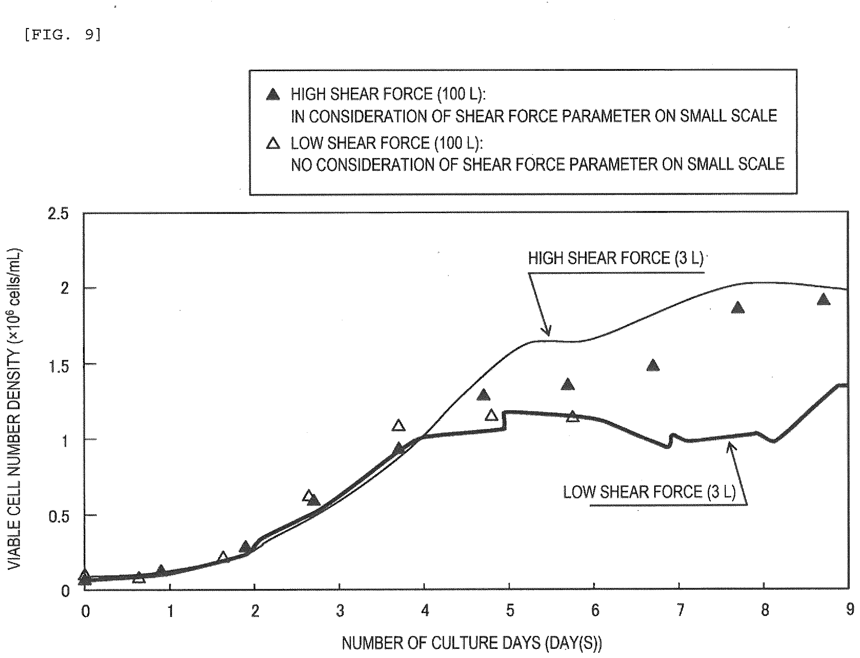

Based on these, a model including a shear force as a parameter was constructed, stirring conditions in 100 L culture under each condition were determined, and the culture was performed. The results are illustrated in FIG. 9. FIG. 9 is a graph illustrating a relationship between the number of culture days and the viable cell number density, and illustrates cell growth curves before the scale-up (3 L) and after the scale-up (100 L). As illustrated in FIG. 9, in the design in consideration of the shear force (that is, the high shear force (100 L): in consideration of the shear force parameter in the small scale), the cell number increased in both of 3 L and 100 L with respect to the low shear force (no consideration of the shear force parameter in the small scale). In addition, according to the model in consideration of the shear force, the same culture results were obtained at 3 L and 100 L in both the high shear force and the low shear force. In other words, a thin solid line with a high shear force (3 L) and ▲ showed the same culture result, and a thick solid line with a low shear force (3 L) and △ showed the same culture result.

In the design according to the related art, the culture process is optimized including the low shear, and the culture tank after scale-up tends to have a high shear, and therefore, there is a high possibility that the cell growth curve has changed.

By using the present method and the present device 1, the same culture result can be obtained before and after scale-up, and the productivity can be improved as compared with a design according to the related art, in which a shear force (second explanatory variable, second function) is not considered.

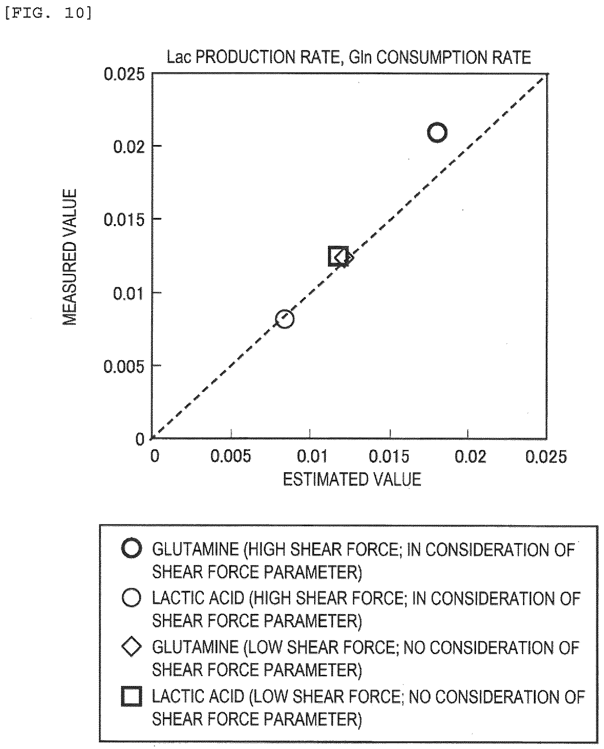

FIG. 10 is a graph illustrating a relationship between measured values of a lactic acid (Lac) production rate and a glutamine (Gln) consumption rate and the estimated values (models) obtained by calculation based on the shear force distribution in the previous section when 100 L culture was performed. In the graph of FIG. 10, a diagonal line indicated by a broken line indicates that the measured value matches the estimated value. That is, the closer the measured value is plotted to the diagonal line, the more the measured value matches the estimated value. As illustrated in FIG. 10, in the present experiment, the measured values are plotted substantially on the diagonal line indicated by the broken line, and it can be seen that the measured values substantially match the estimated values. From this, it was found that by using the present method and the present device 1, it is possible to estimate what value the cell metabolism takes when the scale-up is performed. From this, it was found that the present method and the present device 1 can prevent unexpected problems in productivity and quality after the scale-up.

Although the culture scale-up culture data collection device 1 and the culture scale-up culture data collection method according to the invention have been described above in detail with reference to the embodiments and Examples, the invention is not limited to the embodiments and Examples described above, and includes various modifications. For example, the above embodiments are described in detail to facilitate understanding of the invention, and the invention is not necessarily limited to those including all the configurations described above. A part of a configuration of a certain embodiment can be replaced with a configuration of another embodiment, and a configuration of another embodiment can be added to a configuration of a certain embodiment. Another configuration can be added to, deleted from, or replaced with a part of a configuration of each embodiment.

Reference Signs List

| | |
|---|---|
| 1: | culture scale-up culture data collection device (present device) |
| 2: | culture tank |
| 21: | culture solution |
| 22: | sparger |
| 3: | addition tube |
| 4: | addition pump |
| 5: | medium tank |
| 6: | cell number control unit |
| 6A: | cell bleeding device |
| 6B: | cell separation device |
| 7: | nutrient component concentration control unit |
| 71: | sampling tube |
| 8: | shear force control unit |
| 81: | stirring blade |
| 82: | motor |

S1:    selection step
S2:    collection step
S3:    construction step
S4:    design step
S5:    checking step

**Claims**

1.  A culture scale-up culture data collection device for searching for a scale-up condition of a cell culture device, the device comprising:

    a first function related to a first explanatory variable associated with the cell culture device after scale-up; and
    a second function related to a second explanatory variable, which correlates with an objective variable but is not associated with the cell culture device after the scale-up, wherein
    both the first function and the second function are optimized for the objective variable.

2.  The culture scale-up culture data collection device according to claim 1, wherein

    the first function is a control device that controls at least one of a temperature, a pH, and a dissolved oxygen concentration, and
    the second function is a control device that controls at least one of a shear force, a nutrient component concentration, and a waste product concentration.

3.  The culture scale-up culture data collection device according to claim 2, further comprising:
    at least one of a cell separation device, a cell bleeding device, a medium component analysis device, a medium addition device that performs feedback control based on a value of a medium component, and a high shear force stirring blade, in order to control the second function.

4.  The culture scale-up culture data collection device according to claim 3, wherein
    the cell separation device is a gravity sedimentation type or a hollow fiber membrane separation type.

5.  The culture scale-up culture data collection device according to claim 3, wherein
    the medium addition device includes a Raman spectroscopic sensor, an operation device, and a pump for medium addition.

6.  The culture scale-up culture data collection device according to claim 2, wherein
    a target having the nutrient component concentration to be measured is at least one of glucose, glutamine, and glutamic acid.

7.  The culture scale-up culture data collection device according to claim 2, wherein
    a target having the waste product concentration to be measured is at least one of lactic acid and ammonia.

8.  The culture scale-up culture data collection device according to claim 2, wherein
    at least one of the nutrient component concentration, the waste product concentration, and cell number is controlled to a constant state.

9.  The culture scale-up culture data collection device according to claim 1, further comprising:
    a data collection device configured to collect data related to the first function and data related to the second function.

10. A culture scale-up culture data collection method for searching for a scale-up condition of a cell culture device, the method comprising:

    a selection step of selecting an objective variable, a first explanatory variable associated with the cell culture device after scale-up, and a second explanatory variable that correlates with the objective variable but is not associated with the cell culture device after the scale-up;
    a collection step of collecting data of at least one of a shear force, a nutrient component concentration, a waste product concentration, and a viable cell number concentration by performing a cell culture experiment based on the selected first explanatory variable and the selected second explanatory variable;

a construction step of constructing a model for the scale-up based on the collected data; and
a design step of designing a cell culture tank by evaluating at least one of productivity and quality after the scale-up using the constructed model and fluid analysis.

11. The culture scale-up culture data collection method according to claim 10, further comprising:
after the design step, a checking step of checking by the cell culture experiment whether a desired performance is satisfied, by constructing the cell culture tank designed in the design step.

[FIG. 1A]

[FIG. 1B]

[FIG. 2]

[FIG. 3]

OPTIMIZATION

FIRST EXPLANATORY
VARIABLE
PARAMETERS
STUDIED IN PROCESS
OPTIMIZATION
IN RELATED ART

CORRELATION (1)

CULTURE ENVIRONMENT
VARIABLE (1)
·FLUID    ·CELL STATE
·CULTURE SOLUTION

CORRELATION (2)

OBJECTIVE
VARIABLE
·QUALITY
CHARACTERISTIC
·PRODUCTIVITY

SECOND
EXPLANATORY
VARIABLE
PARAMETER SPECIFIC
TO SCALE-UP

CULTURE ENVIRONMENT
VARIABLE (2)
·FLUID    ·CELL STATE
·CULTURE SOLUTION

EXPERIMENTAL DEVICE

FIRST FUNCTION ASSOCIATED WITH DEVICE AFTER SCALE-UP (CULTURE TANK IN RELATED ART)

SECOND FUNCTION NOT ASSOCIATED WITH DEVICE AFTER SCALE-UP (NEWLY ADDED CULTURE TANK)

[FIG. 4A]

[FIG. 4B]

[FIG. 5]

```
┌─────────────────────────────┐
│   SELECT OBJECTIVE VARIABLE  │
│   AND EXPLANATORY VARIABLE   │─ S1
│      (SELECTION STEP)        │
└─────────────────────────────┘
              │
              ▼
┌─────────────────────────────┐
│        COLLECT DATA          │
│    OF CULTURE EXPERIMENT     │─ S2
│      (COLLECTION STEP)       │
└─────────────────────────────┘
              │
              ▼
┌─────────────────────────────┐
│      CONSTRUCT MODEL         │─ S3
│    (CONSTRUCTION STEP)       │
└─────────────────────────────┘
              │
              ▼
┌─────────────────────────────┐
│         DESIGN TANK          │─ S4
│       (DESIGN STEP)          │
└─────────────────────────────┘
              │
              ▼
 ┌─────────────────────────────┐
(│   CHECK CULTURE IN CULTURE   │     )
 │   TANK AFTER SCALE-UP        │─ S5
 │      (CHECKING STEP)         │
 └─────────────────────────────┘
```

[FIG. 6]

LIQUID SURFACE VENTILATION CONTROL

N₂ (300 mL/min)

CO₂ (APPROPRIATE)

O₂ or Air

PURE OXYGEN VENTILATION (HOLE DIAMETER 10 μm): 22 mL/h
PURE OXYGEN VENTILATION (HOLE DIAMETER 100 μm): 27 mL/h
Air VENTILATION (HOLE DIAMETER 100 μm): 83 mL/h

IN-LIQUID VENTILATION CONTROL

DISCHARGED GAS

CULTURE CONTROLLER

2

21

22

MAGNETIC STIRRER

EP 4 692 298 A1

[FIG. 7]

Figure 7: Graph of VIABLE CELL NUMBER DENSITY (×10⁶ cells/mL) versus NUMBER OF CULTURE DAYS (DAY(S)).

Legend:
- ◆ LIQUID SURFACE VENTILATION
- △ PURE OXYGEN IN-LIQUID VENTILATION (HOLE DIAMETER 100 μm)
- □ PURE OXYGEN IN-LIQUID VENTILATION (HOLE DIAMETER 10 μm)
- ○ Air IN-LIQUID VENTILATION (HOLE DIAMETER 100 μm)

[FIG. 8]

[FIG. 9]

▲ HIGH SHEAR FORCE (100 L):
   IN CONSIDERATION OF SHEAR FORCE PARAMETER ON SMALL SCALE

△ LOW SHEAR FORCE (100 L):
   NO CONSIDERATION OF SHEAR FORCE PARAMETER ON SMALL SCALE

HIGH SHEAR FORCE (3 L)

LOW SHEAR FORCE (3 L)

VIABLE CELL NUMBER DENSITY ($\times 10^6$ cells/mL)

NUMBER OF CULTURE DAYS (DAY(S))

EP 4 692 298 A1

[FIG. 10]

Lac PRODUCTION RATE, Gln CONSUMPTION RATE

○ GLUTAMINE (HIGH SHEAR FORCE; IN CONSIDERATION OF SHEAR FORCE PARAMETER)

○ LACTIC ACID (HIGH SHEAR FORCE; IN CONSIDERATION OF SHEAR FORCE PARAMETER)

◇ GLUTAMINE (LOW SHEAR FORCE; NO CONSIDERATION OF SHEAR FORCE PARAMETER)

□ LACTIC ACID (LOW SHEAR FORCE; NO CONSIDERATION OF SHEAR FORCE PARAMETER)

## INTERNATIONAL SEARCH REPORT

International application No.

**PCT/JP2024/005555**

### A. CLASSIFICATION OF SUBJECT MATTER

*C12M 1/00*(2006.01)i; *C12Q 1/02*(2006.01)i

FI: C12M1/00 C; C12Q1/02

According to International Patent Classification (IPC) or to both national classification and IPC

### B. FIELDS SEARCHED

Minimum documentation searched (classification system followed by classification symbols)

C12M1/00; C12Q1/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2022-531464 A (ZYMERGEN INC.) 06 July 2022 (2022-07-06) claims, paragraphs [0014]-[0016], [0148] | 1-2, 6-11 |
| Y | | 3-5 |
| Y | JP 2018-161115 A (HITACHI, LTD.) 18 October 2018 (2018-10-18) claims, paragraph [0003] | 3-5 |
| Y | JP 2020-195370 A (REGENERON PHARMACEUTICALS, INC.) 10 December 2020 (2020-12-10) claim 1 | 5 |
| A | JP 2018-161057 A (HITACHI, LTD.) 18 October 2018 (2018-10-18) | 1-11 |

☐ Further documents are listed in the continuation of Box C.    ☑ See patent family annex.

| | | |
|---|---|---|
| * | Special categories of cited documents: | |
| "A" | document defining the general state of the art which is not considered to be of particular relevance | |
| "D" | document cited by the applicant in the international application | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **11 March 2024** | **26 March 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/005555**

| Patent document cited in search report | | | Publication date (day/month/year) | Patent family member(s) | | | Publication date (day/month/year) |
|---|---|---|---|---|---|---|---|
| JP | 2022-531464 | A | 06 July 2022 | US claims, paragraphs [0014]-[0015], [0169] | 2022/0328128 | A1 | |
| | | | | EP | 3966822 | A1 | |
| | | | | CN | 113795885 | A | |
| | | | | KR | 10-2022-0006066 | A | |
| JP | 2018-161115 | A | 18 October 2018 | (Family: none) | | | |
| JP | 2020-195370 | A | 10 December 2020 | US claim 1 | 2019/0112569 | A1 | |
| | | | | EP | 3698125 | A1 | |
| | | | | CN | 111201434 | A | |
| | | | | KR | 10-2020-0070218 | A | |
| JP | 2018-161057 | A | 18 October 2018 | WO | 2018/173463 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022531464 A